# EUROPÄISCHE PATENTSCHRIFT

(11) **EP 1 150 660 B1**
(45) Veröffentlichungstag und Bekanntmachung des Hinweises auf die Patenterteilung: **21.01.2004**
(21) Anmeldenummer: 00906318.1
(22) Anmeldetag: 10.02.2000
(51) Int. Cl.: A61K 9/46, A61K 31/4152, A61P 29/00

(54) **PHARMAZEUTISCHE, METAMIZOL ENTHALTENDE BRAUSEFORMULIERUNG**
PHARMACEUTICAL EFFERVESCENT FORMULATION CONTAINING METAMIZOL
FORMULATION EFFERVESCENTE PHARMACEUTIQUE CONTENANT DU METAMIZOL

(30) Priorität: 11.02.1999 DE 19905581
(43) Veröffentlichungstag der Anmeldung: 07.11.2001
(73) Patentinhaber: HEXAL AG, D-83607 Holzkirchen (DE)
(72) Erfinder: FREUDENSPRUNG, Brigitte, Hexal AG, D-83607 Holzkirchen (DE)
(74) Vertreter: Ritthaler, Wolfgang, Dr.rer.nat.Dipl.-Chem.
(86) Internationale Anmeldenummer: PCT/EP2000/001096
(87) Internationale Veröffentlichungsnummer: WO 2000/047189

(56) Entgegenhaltungen:
- EP-A- 0 637 448
- WO-A-98/47534
- WO-A-99/59553
- DE-A- 19 822 036

## Beschreibung

Die Erfindung betrifft eine stabile pharmazeutische Brauseformulierung mit Metamizol in Form seiner pharmazeutisch unbedenklichen Salze als aktivem Bestandteil, wobei der pH-Wert der entstehenden Lösung sauer ist.

Metamizol, N-Methyl-N-(2,3-dimethyl-5-oxo)-1-phenyl-3-pyrazolin-4-yl) aminomethansulfonsäure, stellt ein antipyretisch wirkendes Analgetikum dar. Die analgetische Wirkung erfolgt durch eine Dämpfung der zentralen Schmerzperzeption infolge einer Aktivierung von Neuronen im schmerzhemmenden System. Die Senkung der erhöhten Körpertemperatur wird durch den Angriff am hypothalamischen Wärmeregulationszentrum vermittelt, in dessen Folge eine vermehrte Wärmeabgabe über die Peripherie auftritt.
Nach oraler Zufuhr geht der Resorption von Metamizol eine Hydrolyse zu 4-Methylaminoantipyrin in der Leber voraus. 4-Methylaminoantipyrin und das daraus entstehende 4-Aminoantipyrin sind die pharmakologisch aktiven Metaboliten des Metamizol, während die weiteren Metaboliten 4-Acetylaminoantipyrin (Hauptmetabolit im Urin) und 4-Formylaminoantipyrin als pharmakologisch sehr viel schwächer wirksam oder inaktiv gelten. Nach oraler Applikation von bisher auf dem Markt befindlichen Tabletten wird die pharmakologisch wirksame Plasmakonzentration von 4-Methylaminoantipyrin nach 1,4 Stunden und von 4-Aminoantipyrin nach 6,7 Stunden erreicht.

Die möglichst schnelle Erreichung einer pharmakologisch wirksamen Plasmakonzentration und somit einer therapeutischen Wirkung wird erwünscht, da Metamizol zur Behandlung von akuten starken Schmerzen dient. Die für diese Zwecke oft verwendete Darreichungsform der Injektion bedarf einer nur durch ärztliches Fachpersonal gewährleisteten Betreuung, da sonst bei zu schneller Injektionsgeschwindigkeit und zu hoher Dosierung lebensgefährliche Nebenwirkungen, wie z.B. plötzliches Kreislaufversagen oder Agranulozytose, die Folge sind. Außerdem ist die Patienten-Compliance einer Injektion sehr gering.

Die EP-A-0 637 448 beschreibt pharmazeutische Zusammensetzungen in verschiedenen zur oralen Verabreichung geeigneten Formen, die Glycinamid oder N-Acetylglycinamid zusammen mit einem Analgetikum wie Metamizol enthalten. Metamizol-haltige Brauseformulierugen werden nicht beschrieben.

Die WO 98/47534 betrifft stabilisierte Arzneimittel, die Cysteinylderivate wie N-Acetylcystein enthalten. Diese Arzneimittel können in verschiedenen Darreichungsformen vorliegen und gegebenenfalls auch nichtsteroidale Analgetika wie Metamizol enthalten. Auch hier werden keine Brauseformulierugen für Metamizol beschrieben.

Die ältere WO 99/59553 beschreibt ein Verfahren zur Herstellung von Brausezubereitungen, die pharmazeutische Wirkstoffe wie Metamizol enthalten können, wobei der Brausesatz oder Komponenten davon in geschmolzenem Zucker, Zuckeralkohol oder Zucker-Ersatzstoff als Matrix dispergiert werden.

Die Aufgabe der Erfindung ist es nun, eine stabile Brauseformulierung mit Metamizol und/oder dessen pharmazeutisch unbedenklichen Salzen als aktive Komponenten bereitzustellen, so daß die pharmakologisch wirksame Plasmakonzentration und damit die therapeutische Wirkung schneller als mit den herkömmlichen oral zu verabreichenden Darreichungsformen erreicht, aber auf eine direkte ärztliche Betreuung während der Einnahme verzichtet werden kann, da die Gefahr der lebensgefährlichen Nebenwirkungen nicht gegeben ist.

Die Stabilität von Metamizol bzw. dessen pharmazeutisch unbedenklichen Salzen ist pHabhängig. Im neutralen und basischen Milieu sind Metamizol bzw. dessen pharmazeutisch unbedenklichen Salze stabil. Im sauren Milieu allerdings erfolgt sehr rasch eine Hydrolyse, die sich durch eine Gelbfärbung zeigt. Die zur Herstellung von Brausetabletten üblicherweise verwendeten Hilfsstoffe erzeugen in wässriger Lösung ein saures Milieu.

Es wurde nun überraschenderweise gefunden, daß trotz eines sauren pH-Wertes der Brauseformulierungslösung der Wirkstoff Metamizol bzw. dessen pharmazeutisch unbedenklichen Salze ohne Zusatz von speziellen stabilisierenden Substanzen nicht der Hydrolyse unterliegen, das heißt stabil sind, und sehr schnell ein therapeutisch wirksamer Plasmaspiegel erreicht wird. Zudem besteht gegenüber den bisher auf dem Markt befindlichen oral zu verabreichenden Darreichungsformen der Vorteil, daß durch eine Aromatisierung der Brauseformulierung die Medikamenteneinnahme für den Patienten angenehmer gestaltet wird.

Die erfindungsgemäße Brauseformulierung enthält die pharmazeutisch unbedenklichen Salze von Metamizol als aktive Bestandteile, nämlich Alkalisalze, wie z.B. das Kalium-, Natriumund Lithiumsalz, insbesondere das Metamizol-Natrium-Monohydrat, und/oder das Ammoniumsalz.

Die erfindungsgemäße Brauseformulierung kann pro Dosierungseinheit eine wirksame Menge der pharmazeutisch unbedenklichen Salze von Metamizol von 200-1000 mg, insbesondere von 400-600 mg enthalten.
Die bevorzugte erfindunasgemäße Brauseformulierung enthält 500 mg des Metamizol-Natrium-Monohydrats pro Dosierungseinheit.

Die erfindungsgemäße Brauseformulierung, gelöst in Wasser, weist einen pH-Wert von 3 bis 6,5, insbesondere von 4 bis 6, bevorzugt von 4,5-5 auf und die resultierende Lösung ist mindestens über eine Stunde stabil und klar.

In der erfindungsgemäßen Brauseformulierung kann als Kohlendioxidquelle das Carbonat und/ oder das Bicarbonat der Alkali- und/ oder der Erdalkalimetalle, z.B. Natriumcarbonat bzw. Natriumbicarbonat, Calciumcarbonat bzw. Calciumbicarbonat und/ oder Magnesiumcarbonat bzw. Magnesiumbicarbonat, in Verbindung mit mindestens einer Säure, z.B. Citronensäure, Mononatriumcitrat, Ascorbinsäure, Gluconsäure, Milchsäure, Äpfelsäure und Weinsäure, verwendet werden. In der bevorzugten erfindungsgemäßen Brauseformulierung wird Citronensäure mit einer Kombination von Natriumcarbonat und Natriumbicarbonat als Brausegemisch verwendet. Dabei kann das Gewichtsverhältnis von der oder den Säure(n) zu dem Carbonat und/ oder dem Bicarbonat zwischen 0,7 und 2,4, insbesondere zwischen 1,1 und 2,2 liegen.

Ein akzeptabler Geschmack der resultierenden Lösung des Wirkstoffes kann durch Zusatz geeigneter Geschmackskorrigenten, wie Aromastoffen, Zucker, Saccharose bzw. Zuckeraustaustauschstoffen erreicht werden. Zucker oder Zuckeraustauschstoffe können in einer Menge von bis zu etwa 50 Gew.% vorliegen. Zur Herstellung der erfindungsgemäßen Brauseformulierung geeignete Geschmackskorrigenten sind künstliche oder natürliche Süßstoffe (0,5 bis 5 Gew.%), vorzugsweise Saccharin-Natrium, Natrium-Cyclamat, Sorbit, Aspartam oder Mannit, oder künstliche oder natürliche Aromastoffe, vorzugsweise Zitronen-, Banane-, Pfefferminze-, Karamel-, Waldfrucht- und Himbeeraroma. Die Aromen werden vorzugsweise in einer Menge von 0,5 bis 3 Gew.% verwendet.

Weiterhin werden nach dem Stand der Technik bekannte, pharmazeutische, wasserlösliche Tablettenhilfsstoffe zur Herstellung der erfindungsgemäßen Brauseformulierung, z.B. Füllund Bindemittel (Mannit) sowie Schmiermittel (Polyethylenglykole, Compritol, L-Leucin, Magnesiumstearat, Stearinsäure) eingesetzt. Als weitere mögliche Tablettenhilfsstoffe können gegebenenfalls ein oder mehrere Polysaccharide zugegeben werden. Bevorzugte Polysaccharide sind Cyclodextrine. Mögliche Vertreter stellen α-, β-, γ- Cyclodextrin und/oder deren pharmazeutisch unbedenklichen Derivate, insbesondere β-Cyclodextrin, dar.

Die erfindungsgemäße Brauseformulierung kann in Form von Pulver, Tabletten oder Granulat, welches in Sachets abgefüllt werden kann, vorliegen. Die bevorzugte Brauseformulierung liegt in Form von Tabletten vor.

Die Erfindung wird durch nachstehende Beispiele näher erläutert ohne aber den Erfindungsumfang damit einzuschränken.

### Beispiel 1:

| | |
|---|---|
| Metamizol-Natrium-Monohydrat | 500 mg/ Tabl. |
| Citronensäure | 1230 mg/ Tabl. |
| Natriumcarbonat | 650 mg/ Tabl. |
| Natriumhydogencarbonat (Bicarbonat) | 75 mg/ Tabl. |
| Ascorbinsäure | 75 mg/ Tabl. |
| Lactose | 500 mg/ Tabl. |
| Saccharin-Natrium | 5 mg/ Tabl. |
| PEG 6000 | 155 mg/ Tabl. |
| Natrium-Cyclamat | 50 mg/ Tabl. |
| Aroma (Zitrone) | 50 mg/ Tabl. |

Das Gesamtgewicht einer Tablette beträgt 3290 mg.
Metamizol-Natrium-Monohydrat und PEG 6000 werden gemahlen und gesiebt, die restlichen Bestandteile werden zugemischt. Das Gemisch wird zu Tabletten mit 20 mm Durchmesser und 3290 mg verpreßt. Nach dem Auflösen resultiert eine klare Lösung. Der Wirkstoff ist über einer Stunde stabil. Das Gewichtsverhältnis von Citronensäure zu Carbonat/Bicarbonat entspricht ca. 1,7.

### Beispiel 2:

| | |
|---|---|
| Metamizol-Natrium-Monohydrat | 500 mg/ Tabl. |
| Citronensäure | 1230 mg/ Tabl. |
| Natriumcarbonat | 650 mg/ Tabl. |
| Natriumhydogencarbonat (Bicarbonat) | 75 mg/ Tabl. |
| Ascorbinsäure | 75 mg/ Tabl. |
| Lactose-Monohydrat | 500 mg/ Tabl. |
| Saccharin-Natrium | 5 mg/ Tabl. |
| PEG 6000 | 155 mg/ Tabl. |
| Natrium-Cyclamat | 50 mg/ Tabl. |
| Aroma (Himbeere) | 30 mg/ Tabl. |

Das Gesamtgewicht einer Tablette beträgt 3270 mg.
Metamizol-Natrium-Monohydrat und PEG 6000 werden gemahlen und gesiebt, die restlichen Bestandteile werden zugemischt. Das Gemisch wird zu Tabletten mit 25 mm Durchmesser und 3270 mg mit Hilfe einer Rundlauftablettenmaschine verpreßt. Nach dem Auflösen resultiert eine klare Lösung. Der Wirkstoff ist über einer Stunde stabil. Das Gewichtsverhältnis von Citronensäure zu Carbonat/Bicarbonat entspricht ca. 1,7.

### Beispiel 3:

| | |
|---|---|
| Metamizol-Natrium-Monohydrat | 500 mg/ Tabl. |
| Citronensäure | 1500 mg/ Tabl. |
| Natriumcarbonat wasserfrei | 630 mg/Tabl. |
| Natriumhydogencarbonat (Bicarbonat) | 70 mg/ Tabl. |
| PEG 6000 | 150 mg/Tabl. |
| Saccharin-Natrium | 5 mg/ Tabl. |
| Natrium-Cyclamat | 50 mg/Tabl. |
| Ascorbinsäure | 75 mg/Tabl. |
| Lactose | 820 mg/Tabl. |
| Aroma (Himbeere) | 30 mg/ Tabl. |

Das Gesamtgewicht einer Tablette beträgt 3830 mg.
Metamizol-Natrium-Monohydrat und PEG 6000 werden gemahlen und gesiebt, die restlichen Bestandteile werden zugemischt. Das Gemisch wird zu Tabletten mit 22 mm Durchmesser und 3830 mg verpreßt. Das Gewichtsverhältnis von Citronensäure zu Carbonat/Bicarbonat entspricht ca. 2,14. Der pH-Wert der Lösung beträgt 4,50-4,61.

### Beispiel 4:

| | |
|---|---|
| Metamizol-Natrium-Monohydrat | 500 mg/ Tabl. |
| Citronensäure | 750 mg/ Tabl. |
| Natriumcarbonat wasserfrei | 900 mg/Tabl. |
| Natriumhydogencarbonat (Bicarbonat) | 50 mg/ Tabl. |
| Mannit | 30 mg/Tabl. |
| β-Cyclodextrin | 100 mg/Tabl. |
| PEG 6000 | 96 mg/Tabl. |
| Saccharin-Natrium | 4 mg/ Tabl. |
| Natrium-Cyclamat | 15 mg/Tabl. |
| Ascorbinsäure | 75 mg/Tabl. |
| Lactose und PVP | 80 mg/Tabl. |
| Aroma (Zitrone) | 50 mg/ Tabl. |

Das Gesamtgewicht einer Tablette beträgt 2650 mg.
Metamizol-Natrium-Monohydrat, Mannit und PEG 6000 werden gemahlen und gesiebt, die restlichen Bestandteile werden zugemischt. Das Gemisch wird zu Tabletten mit 22 mm Durchmesser und 2650 mg verpreßt. Der pH-Wert der Lösung beträgt ca. 5. Das Gewichtsverhältnis von Citronensäure zu Carbonat/Bicarbonat entspricht ca. 0,79.

### Beispiel 5:

| | |
|---|---|
| Metamizol-Natrium-Monohydrat | 500 mg/ Tabl. |
| Citronensäure | 1200 mg/ Tabl. |
| Natriumcarbonat wasserfrei | 600 mg/Tabl. |
| Natriumhydogencarbonat (Bicarbonat) | 118 mg/ Tabl. |
| PEG 6000 | 350 mg/Tabl. |
| Saccharin-Natrium | 4 mg/ Tabl. |
| Natrium-Cyclamat | 40 mg/Tabl. |
| Äpfelsäure | 300 mg/Tabl. |
| Lactose | 120 mg/Tabl. |
| Aroma (Himbeere) | 30 mg/ Tabl. |

Das Gesamtgewicht einer Tablette beträgt 3262 mg.
Metamizol-Natrium-Monohydrat und PEG 6000 werden gemahlen und gesiebt, die restlichen Bestandteile werden zugemischt. Das Gemisch wird zu Tabletten verpreßt. Nach dem Auflösen resultiert eine klare Lösung. Der Wirkstoff ist über einer Stunde stabil. Das Gewichtsverhältnis von Citronensäure zu Carbonat/Bicarbonat entspricht ca. 1,67. Der pH-Wert der Lösung beträgt 4,56-4,72.

### Beispiel 6:

| | |
|---|---|
| Metamizol-Natrium-Monohydrat | 500 mg/ Tabl. |
| Citronensäure | 660 mg/ Tabl. |
| Natriumcarbonat wasserfrei | 100 mg/Tabl. |
| Natriumhydogencarbonat (Bicarbonat) | 420 mg/ Tabl. |
| PEG 8000 | 25 mg/Tabl. |
| Saccharin-Natrium | 5 mg/ Tabl. |
| Natrium-Cyclamat | 30 mg/Tabl. |
| Ascorbinsäure | 75 mg/Tabl. |
| Lactose | 120 mg/Tabl. |
| Aroma (Waldbeere/Brombeere) | 50 mg/ Tabl. |

Das Gesamtgewicht einer Tablette beträgt 1985 mg.
Metamizol-Natrium-Monohydrat und PEG 8000 werden gemahlen und gesiebt, die restlichen Bestandteile werden zugemischt. Das Gemisch wird zu Tabletten mit einem Durchmesser von 20 mm verpreßt. Das Gewichtsverhältnis von Citronensäure zu Carbonat/Bicarbonat entspricht ca. 1,27.

### Beispiel 7:

| | |
|---|---|
| Metamizol-Natrium-Monohydrat | 500 mg/Tabl. |
| Citronensäure | 666 mg/Tabl. |
| Natriumcarbonat wasserfrei | 633 mg/Tabl. |
| Natriumhydogencarbonat (Bicarbonat) | 100 mg/Tabl. |
| PEG 6000 | 350 mg/Tabl. |
| Saccharin-Natrium | 4 mg/Tabl. |
| Natrium-Cyclamat | 40 mg/Tabl. |
| Äpfelsäure | 200 mg/Tabl. |
| Lactose | 276 mg/Tabl. |
| Aroma (Himbeere) | 30 mg/ Tabl. |

Das Gesamtgewicht einer Tablette beträgt 2799 mg.
Metamizol-Natrium-Monohydrat und PEG 6000 werden gemahlen und gesiebt, die restlichen Bestandteile werden zugemischt. Das Gemisch wird zu Tabletten verpreßt. Das Gewichtsverhältnis von Citronensäure zu Carbonat/Bicarbonat entspricht ca. 0,91. Der pH-Wert der Lösung beträgt 5,73-5,92.

## Patentansprüche

1. Pharmazeutische Zusammensetzung in Form einer Brauseformulierung, welche ein oder mehrere Alkaliund/oder Ammoniumsalze von Metamizol als aktiven Bestandteil enthält.

2. Pharmazeutische Zusammensetzung in Form einer Brauseformulierung nach Anspruch 1, welche Metamizol-Natrium-Monohydrat als aktiven Bestandteil enthält.

3. Pharmazeutische Zusammensetzung in Form einer Brauseformulierung nach Anspruch 1 oder 2, wobei der Gehalt an Alkali- und/oder Ammoniumsalz von Metamizol 200-1000 mg, insbesondere 400-600 mg pro Dosierungseinheit beträgt.

4. Pharmazeutische Zusammensetzung in Form einer Brauseformulierung nach Anspruch 3, wobei der Gehalt an Metamizol-Natrium-Monohydrat 500 mg pro Dosierungseinheit beträgt.

5. Pharmazeutische Zusammensetzung in Form einer Brauseformulierung nach einem der Ansprüche 1-4, welche mindestens eine physiologisch verträgliche Säure oder deren Natriumsalz und ein physiologisch verträgliches Carbonat und/oder Bicarbonat in solchen Mengenverhältnissen als Brausegemisch enthält, daß die resultierende Lösung einen pH-Wert von 3 bis 6,5, insbesondere von 4 bis 6, bevorzugt von 4,5 bis 5 aufweist.

6. Pharmazeutische Zusammensetzung in Form einer Brauseformulierung nach Anspruch 5, welche Citronensäure oder deren Mononatriumsalz in Kombination mit Natriumcarbonat und Natriumbicarbonat als Brausegemisch enthält.

7. Pharmazeutische Zusammensetzung in Form einer Brauseformulierung nach einem der Ansprüche 1-6, welche einen Süßstoff enthält.

8. Pharmazeutische Zusammensetzung in Form einer Brauseformulierung nach einem der Ansprüche 1-7, welche einen Aromastoff enthält.

9. Pharmazeutische Zusammensetzung in Form einer Brauseformulierung nach einem der Ansprüche 1-8 in Form von Pulver, Tabletten oder Granulat, welches in Sachets abgefüllt werden kann, insbesondere in Form von Tabletten.

10. Pharmazeutische Zusammensetzung nach einem der Ansprüche 1-9, wobei die Alkali- und/oder Ammoniumsalze von Metamizol als aktive Bestandteile in der Brauseformulierungslösung mindestens eine Stunde stabil sind, d.h. nicht der Hydrolyse unterliegen.

## Claims

1. Pharmaceutical composition in the form of an effervescent formulation, containing one or several alkali and/or ammonium salts of Metamizol as the active constituent.

2. Pharmaceutical composition in the form of an effervescent formulation according to Claim 1, containing Metamizol Sodium Monohydrate as the active constituent.

3. Pharmaceutical composition in the form of an effervescent formulation according to Claim 1 or 2, whereby the content of alkali and/or ammonium salt of Metamizol amounts to 200 - 1000 mg, in particular 400 - 600 mg per dose unit.

4. Pharmaceutical composition in the form of an effervescent formulation according to Claim 3, whereby the content of Metamizol Sodium Monohydrate amounts to 500 mg per dose unit.

5. Pharmaceutical composition in the form of an effervescent formulation according to one of the Claims 1 - 4, which contains at least one physiologically compatible acid or its sodium salt and a physiologically compatible carbonate and/or bicarbonate as an effervescent mixture in quantitative ratios such that the resulting solution has a pH from 3 to 6.5, in particular from 4 to 6 and preferably from 4.5 to 5.

6. Pharmaceutical composition in the form of an effervescent formulation according to Claim 5, which contains citric acid or its monosodium salt in combination with sodium carbonate and sodium bicarbonate as an effervescent mixture.

7. Pharmaceutical composition in the form of an effervescent formulation according to one of the Claims 1 - 6, containing a sweetener.

8. Pharmaceutical composition in the form of an effervescent formulation according to one of the Claims 1 - 7, containing a flavouring agent.

9. Pharmaceutical composition in the form of an effervescent formulation according to one of the Claims 1 - 8, in the form of powder, tablets or granulate that can be packed into sachets, in particular in the form of tablets.

10. Pharmaceutical composition in the form of an effervescent formulation according to one of the Claims 1 - 9, whereby the alkali and/or ammonium salts of Metamizol as the active constituents are stable in the effervescent formulation solution for at least one hour, i.e. do not undergo hydrolysis.

## Revendications

1. Composition pharmaceutique sous forme d'une formulation effervescente qui contient un ou plusieurs sels alcalins et/ou d'ammonium du métamizol comme constituant actif.

2. Composition pharmaceutique sous forme d'une formulation effervescente selon la revendication 1 qui contient du métamizol sodique monohydraté comme constituant actif.

3. Composition pharmaceutique sous forme d'une formulation effervescente selon la revendication 1 ou 2 où la teneur en sel alcalin et/ou d'ammonium du métamizol est 200-1000 mg, en particulier 400-600 mg par unité de prise.

4. Composition pharmaceutique sous forme d'une formulation effervescente selon la revendication 3 où la teneur en métamizol sodique monohydraté est 500 mg par unité de prise.

5. Composition pharmaceutique sous forme d'une formulation effervescente selon l'une des revendications 1-4 qui contient au moins un acide physiologiquement acceptable ou son sel de sodium et un carbonate et/ou bicarbonate physiologiquement acceptable comme mélange effervescent en des proportions telles que la solution résultante présente un pH de 3 à 6,5, en particulier de 4 à 6, de préférence de 4,5 à 5.

6. Composition pharmaceutique sous forme d'une formulation effervescente selon la revendication 5 qui contient de l'acide citrique ou son sel monosodique en combinaison avec du carbonate de sodium et du bicarbonate de sodium comme mélange effervescent.

7. Composition pharmaceutique sous forme d'une formulation effervescente selon l'une des revendications 1-6 qui contient un édulcorant.

8. Composition pharmaceutique sous forme d'une formulation effervescente selon l'une des revendications 1-7 qui contient une substance aromatisante.

9. Composition pharmaceutique sous forme d'une formulation effervescente selon l'une des revendications 1-8 sous forme de poudre, de comprimés ou de granulés qui peuvent être introduite dans des sachets, en particulier sous forme de comprimés.

10. Composition pharmaceutique selon l'une des revendications 1-9. où les sels alcalins et/ou d'ammonium du métamizol comme constituants actifs dans la solution de formulation effervescente sont stables pendant au moins une heure, c'est à dire ne subissent pas d'hydrolyse.
